# EUROPEAN PATENT APPLICATION

(11) **EP 4 190 325 A1**
(43) Date of publication of application: **07.06.2023**
(21) Application number: 21854525.9
(22) Date of filing: 02.08.2021
(51) Int. Cl.: A61K 31/26, A61K 31/426, A61K 31/44, A61K 31/4965, A61K 31/54, A61P 25/08, A61P 25/18, A61P 25/20, A61P 25/22, A61P 25/24

(54) **SENSORY STIMULATING PSYCHOTROPIC DRUG**

(30) Priority: 03.08.2020 JP 2020131872
(71) Applicant: Kansai Medical University, Hirakata-shi, Osaka, 573-1010 (JP); Scent Science International Inc., Ibaraki-shi, Osaka, 567-0085 (JP)
(72) Inventor: KOBAYAKAWA, Ko, Hirakata-shi, Osaka 573-1010 (JP); KOBAYAKAWA, Reiko, Hirakata-shi, Osaka 573-1010 (JP)
(74) Representative: Heubeck, Christian
(86) International application number: PCT/JP2021/028583
(87) International publication number: WO 2022/030436

(57) **Abstract**

The present invention provides a medicament that exerts a psychotropic action through selective sensory nerve stimulation. A prophylactic or therapeutic agent for a psychiatric disorder, containing, as an active ingredient, at least one kind selected from a heterocyclic compound represented by the formula (I) 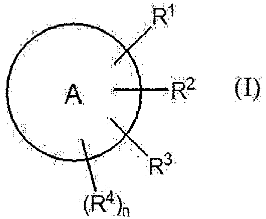 wherein each symbol is as described in the specification, or a salt thereof, and an isothiocyanate compound represented by the formula S=C=N-R⁵ (II) wherein each symbol is as described in the specification.

## Description

### [Technical Field]

The present invention relates to techniques for inducing psychotropic effects by selective sensory nerve stimulation.

### [Background Art]

Human and animal brains have evolved survival skills, such as the ability to recognize and adapt to diversely changing environments, the ability to protect oneself from danger of physical or mental harm, and the ability to establish and maintain appropriate social relationships. These abilities are encoded in the genes as innate survival skills. The brain in a normal state can demonstrate the ability to sustain life efficiently while adapting to the environment. In contrast, a condition in which an abnormal brain condition causes long-term difficulty in adapting to the social environment is considered to be a psychiatric disorder.

Psychiatric disorders are induced by acquired brain changes caused by the single or continuous application of sensory stimulation that affects the emotional system of the brain, such as strong fear and anxiety. For example, in patients with post traumatic stress disorders (PTSD) and after receiving a life-threatening intense fear stimulation, various problematic symptoms persist for a long time due to some acquired change in the brain (Non Patent Literature 1). If there is a technique to restore the acquired changes that cause psychiatric disorders to a normal state, the problematic symptoms are considered to be alleviated. However, no technique currently exists to counteract the acquired brain changes associated with psychiatric disorders.

It is considered that inflammation occurring in the brain is involved in the conditions of psychiatric disorders (Non Patent Literature 2). Inflammation is a response that is essentially necessary for immune cells to destroy exogenous substances that have invaded the body. However, when inflammation progresses excessively, immune cells destroy not only exogenous substances but also their own normal cells. When excessive inflammation occurs in the brain, the cells responsible for brain function are destroyed and normal brain function cannot be exhibited, which is considered to lead to the onset of psychiatric disorders.

There is also known a technique that uses sensory stimulation to promote the treatment of psychiatric disorders, taking advantage of the fact that sensory stimulation can affect the brain system that controls the symptoms of psychiatric disorders. Such techniques include electrical, magnetic, and ultrasonic stimulation of sensory nerves such as vagus nerves, trigeminal nerves, and the like (Non Patent Literature 3). Vagus nerve stimulation is known to have the effect of suppressing inflammation in the body (Non Patent Literature 4). Therefore, vagus nerve stimulation may provide psychotropic actions via anti-inflammatory actions. However, the mechanism by which the sensory nerve stimulation method provides psychotropic action is not known well (Non Patent Literature 5).

It is known that the vagus nerve and trigeminal nerve contain multiple types of nerve cells with different properties, and that the functions of the respective nerve cells are different (Non Patent Literatures 6, 7). Therefore, if it is possible to stimulate only the nerve cells that have the effect of alleviating the symptoms of psychiatric disorders, more efficient treatment methods may be developed. However, it is difficult to differentiate and stimulate different types of nerve cells by physical sensory nerve stimulation methods. If only the appropriate types of nerve cells can be stimulated, sensory information with higher specificity can be transmitted to the brain. As a result, it may be possible to develop more effective treatment techniques for psychiatric disorders.

Sensory nerves can also be stimulated by odor molecules. Aromatherapy is a technique that uses odor molecules. It is considered that aromatherapy exhibits effects when olfactory neuron perceives volatilized odor molecules, or when odor molecules dissolved in essential oils are taken into the body through the skin. Odor components that affect mental conditions are known in aromatherapy (Non Patent Literature 8). However, it is not well understood what receptors the odor molecules used in aromatherapy bind to and through what neural pathways they affect the mental conditions. In addition, the type of odor molecules that have therapeutic effects on psychiatric disorders is not known.

At present, the techniques for inducing psychotropic action by using sensory stimulation have at least two problems. One is that there is no technique to selectively stimulate sensory nerves. The other is that it is unclear what kind of sensory stimulation should be used. If we can overcome these problems and develop a technique to effectively suppress the acquired changes and excessive inflammatory responses in the brain that cause psychiatric disorders, it can be used as a treatment method for psychiatric disorders. However, no technique has heretofore existed that alleviates acquired abnormalities and inflammation in the brain and induces psychotropic action through selective stimulation of sensory nerves.

It is considered that fear emotion evolved as a function of the brain that integrally induces behavioral and physiological responses that increase the chance of survival of individuals in crisis situations. The present inventors formed a hypothesis that, if this is the case, an appropriate type of innate fear stimulation could lead to the induction of latent life-protective actions.

According to this hypothesis, the present inventors found the principle that thiazolines and similar heterocyclic compounds, or isothiocyanate compounds, which are collectively called thiazoline-related fear odors induce latent life-protective actions by intervening in the brain's crisis response system, which integrates and controls the state of the entire body, via the Transient receptor potential ankyrin 1 (Trpa1) gene, which is a sensory receptor on the trigeminal nerve and vagus nerve, and the olfactory receptor genes (Non Patent Literature 9). The life-protective actions that have been clarified to date include induction of hypothermia and hypometabolism, induction of hypoxic resistance, anti-inflammatory action, therapeutic action on wounds, therapeutic action on ischemia reperfusion injury, and the like (Patent Literature 1).

### [Citation List]

### [Patent Literature]

[PTL 1]
WO 2019/177142

### [Non Patent Literature]

[NPL 1]
   Kohji FUKUNAGA et al., Folia Pharmacol. Jpn. 152, 194-201 (2018)
[NPL 2]
   Reus, GZ et al., Neuroscience 300, 141-154 (2015)
[NPL 3]
   Satoshi AYUZAWA et al., Jpn J Neurosurg 26, 864-872 (2017)
[NPL 4]
   Bonaz B et al., J Physiol 594, 5781-5890 (2016)
[NPL 5]
   Nemeroff CB, et al., Neuropsychopharmacology 31, 1345-1355 (2016)
[NPL 6]
   Kupari J et al., Cell Report 27, 2508-2523 (2019)
[NPL 7]
   Lopes DM et al., Frontiers in Mol. Neuro. 10, 304 (2017)
[NPL 8]
   Jiro IMANISHI, Japanese Journal of Complementary and Alternative Medicine 1, 53-61 (2004)
[NPL 9]
   Matsuo T et al., bioRxiv 10.1102/2020.05.17.100933 (2020)

### [Summary of Invention]

### [Technical Problem]

The present invention aims to provide a medicament that exerts a psychotropic action through selective sensory nerve stimulation.

### [Solution to Problem]

Generally, fear and stress stimulation are considered to adversely affect the mind and body. Thiazoline-related fear odors induce innate fear behaviors. However, the present inventors hypothesized that thiazoline-related fear odor stimulation may also have therapeutic effects on psychiatric disorders induced by acquired brain abnormalities and inflammation, if such stimulation induces latent life-protective actions. In order to verify this hypothesis, a thiazoline-related fear odor stimulation was given, and it was clarified that the formation of acquired fear memory was strongly inhibited, and the inflammatory action in the brain was suppressed.

Therefore, the present invention has developed a technique for inducing a psychotropic action by using thiazoline-related fear odors and according to the principle of selectively activating sensory nerves and inducing latent life-protective abilities.

That is, the present invention relates to the following.
[1] A prophylactic or therapeutic agent for a psychiatric disorder, comprising, as an active ingredient, at least one kind selected from a heterocyclic compound represented by the formula (I) 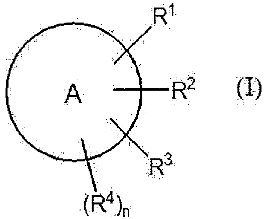 wherein
   ring A is a 5- to 7-membered heterocycle containing 1 or 2 hetero atoms selected from a nitrogen atom, an optionally oxidized sulfur atom and an oxygen atom;
   R¹, R², R³ and R⁴ are each independently a hydrogen atom, a C₁₋₆ alkyl group, a C₁₋₆ alkoxy group, a halogen atom, an amino group, -SH, a C₁₋₆ alkylthio group, a C₂₋₆ alkenylthio group, a C₁₋₆ alkyl-carbonyl group, a formyl group, a C₆₋₁₀ aryl group, a C₁₋₆ alkoxycarbonyl group, a 5- or 6-membered heteroaryl group, or an oxo group; R¹ and R² are optionally bonded to each other to form an optionally substituted 5- or 6-membered ring; and n is 0, 1, or 2
   or a salt thereof, and
   an isothiocyanate compound represented by the formula (II)

      S=C=N-R⁵ (II)

      wherein R⁵ is a C₁₋₆ alkyl group, a C₁₋₆ haloalkyl group, a C₂₋₆ alkenyl group, a C₆₋₁₀ aryl group, or a 5- or 6-membered heteroaryl group.
[2] The agent of [1], wherein the ring A is thiazoline, thiazole, thiazolidine, thiomorpholine, thiophene, pyrrole, morpholine, azepane, pyridine, pyrazine, furan, 2,3-dihydro-4H-1,4-thiazine, or imidazole.
[3] The agent of [1] or [2], wherein the active ingredient is a heterocyclic compound represented by the formula (I) or a salt thereof.
[4] The agent of [1], wherein the active ingredient is an isothiocyanate compound represented by the formula (II).
[5] The agent of any one of [1] to [4], wherein the psychiatric disorder is selected from a post traumatic stress disorder, depression, a panic disorder, an anxiety disorder, a dissociative disorder, an obsessive-compulsive disorder, an eating disorder, a sleep disorder, a bipolar disorder, an adjustment disorder, schizophrenia, and epilepsy.
[6] The agent of any one of [1] to [5], which is for intranasal administration.
[7] Use of at least one kind of compound selected from a heterocyclic compound represented by the formula (I) or a salt thereof, and an isothiocyanate compound represented by the formula (II) for the production of a prophylactic or therapeutic agent for a psychiatric disorder.
[8] The use of [7], wherein the ring A is thiazoline, thiazole, thiazolidine, thiomorpholine, thiophene, pyrrole, morpholine, azepane, pyridine, pyrazine, furan, 2,3-dihydro-4H-1,4-thiazine, or imidazole.
[9] The use of [7] or [8], wherein the aforementioned compound is a heterocyclic compound represented by the formula (I) or a salt thereof.
[10] The use of [7], wherein the aforementioned compound is an isothiocyanate compound represented by the formula (II).
[11] The use of any one of [7] to [10], wherein the psychiatric disorder is selected from a post traumatic stress disorder, depression, a panic disorder, an anxiety disorder, a dissociative disorder, an obsessive-compulsive disorder, an eating disorder, a sleep disorder, a bipolar disorder, an adjustment disorder, schizophrenia, and epilepsy.
[12] The use of any one of [7] to [11], wherein the prophylactic or therapeutic agent is for intranasal administration.
[13] A method for preventing or treating a psychiatric disorder in a mammal, comprising administering an effective amount of at least one kind of compound selected from a heterocyclic compound represented by the formula (I) or a salt thereof, and an isothiocyanate compound represented by the formula (II) to the mammal.
[14] The method of [13], wherein the ring A is thiazoline, thiazole, thiazolidine, thiomorpholine, thiophene, pyrrole, morpholine, azepane, pyridine, pyrazine, furan, 2,3-dihydro-4H-1,4-thiazine, or imidazole.
[15] The method of [13] or [14], wherein the aforementioned compound is a heterocyclic compound represented by the formula (I) or a salt thereof.
[16] The method of [13], wherein the aforementioned compound is an isothiocyanate compound represented by the formula (II).
[17] The method of any one of [13] to [16], wherein the psychiatric disorder is selected from a post traumatic stress disorder, depression, a panic disorder, an anxiety disorder, a dissociative disorder, an obsessive-compulsive disorder, an eating disorder, a sleep disorder, a bipolar disorder, an adjustment disorder, schizophrenia, and epilepsy.
[18] The method of any one of [13] to [17], wherein the aforementioned compound is intranasally administered.
[19] At least one kind of compound selected from a heterocyclic compound represented by the formula (I) or a salt thereof, and an isothiocyanate compound represented by the formula (II) for use in the prophylaxis or treatment of a psychiatric disorder.
[20] The compound for use according to [19], wherein the ring A is thiazoline, thiazole, thiazolidine, thiomorpholine, thiophene, pyrrole, morpholine, azepane, pyridine, pyrazine, furan, 2,3-dihydro-4H-1,4-thiazine, or imidazole.
[21] The compound for use according to [19] or [20], which is a heterocyclic compound represented by the formula (I) or a salt thereof.
[22] The compound for use according to [19], which is an isothiocyanate compound represented by the formula (II).
[23] The compound for use according to any one of [19] to [22], wherein the psychiatric disorder is selected from a post traumatic stress disorder, depression, a panic disorder, an anxiety disorder, a dissociative disorder, an obsessive-compulsive disorder, an eating disorder, a sleep disorder, a bipolar disorder, an adjustment disorder, schizophrenia, and epilepsy.
[24] The compound for use according to any one of [19] to [23], which is for intranasal administration.

### [Advantageous Effects of Invention]

According to the present invention, a prophylactic or therapeutic agent for psychiatric disorders is provided that induces psychotropic actions according to the principle of inducing latent life-protective abilities by selectively activating the sensory nerves. The prophylactic or therapeutic agent for psychiatric disorders of the present invention can be used for the prophylaxis or treatment of psychiatric disorders (e.g., post traumatic stress disorder, depression, panic disorder, anxiety disorder, dissociative disorder, obsessive-compulsive disorder, eating disorder, sleep disorder, bipolar disorder, adjustment disorder, schizophrenia, epilepsy, and the like).

### [Brief Description of Drawings]

[Fig. 1]
   Contextual fear conditioning was performed in an environment with no odor (control) or with thiazoline-related fear odor (2-methyl-2-thiazoline; 2MT), and the freezing behavior of mice was measured the next day in the same context and three days later in the context with another odor (Eugenol), the results of which are shown.
[Fig. 2]
   Contextual fear conditioning was performed 30 min after intraperitoneal injection of thiazoline-related fear odor (tFO) (2-methyl-2-thiazoline, 5-methylthiazole, 4-ethyl-2-methyl-2-thiazoline, 2,4,5-trimethyl-3-thiazoline, thiomorpholine, allyl isothiocyanate (AITC), 2,3-diethylpyrazine, 2,6-lutidine) or saline, and the freezing behavior of mice was measured the next day in the same context, the results of which are shown.
[Fig. 3]
   Contextual fear conditioning was performed 30 min after intraperitoneal injection of 2-(allylthio)-2-thiazoline or saline, and the freezing behavior of mice was measured the next day in the same context, the results of which are shown.
[Fig. 4]
   Contextual fear conditioning was performed 30 min after intraperitoneal injection of 4-tert-butyl-2-methylthiazole or saline, and the freezing behavior of mice was measured the next day in the same context, the results of which are shown.
[Fig. 5]
   Contextual fear conditioning was performed 30 min after intraperitoneal injection of 2-ethoxythiazole or saline, and the freezing behavior of mice was measured the next day in the same context, the results of which are shown.
[Fig. 6]
   Contextual fear conditioning was performed 30 min after intraperitoneal injection of 2-methylbenzothiazole or saline, and the freezing behavior of mice was measured the next day in the same context, the results of which are shown.
[Fig. 7]
   Contextual fear conditioning was performed 30 min after intraperitoneal injection of 2-mercaptothiazole or saline, and the freezing behavior of mice was measured the next day in the same context, the results of which are shown.
[Fig. 8]
   Contextual fear conditioning was performed 30 min after intraperitoneal injection of 2-methylthiomorpholine or saline, and the freezing behavior of mice was measured the next day in the same context, the results of which are shown.
[Fig. 9]
   Contextual fear conditioning was performed 30 min after intraperitoneal injection of 2-acetylpyrrole or saline, and the freezing behavior of mice was measured the next day in the same context, the results of which are shown.
[Fig. 10]
   Contextual fear conditioning was performed 30 min after intraperitoneal injection of 2-methylthiazole or saline, and the freezing behavior of mice was measured the next day in the same context, the results of which are shown.
[Fig. 11]
   Contextual fear conditioning was performed 30 min after intraperitoneal injection of methallyl isothiocyanate or saline, and the freezing behavior of mice was measured the next day in the same context, the results of which are shown.
[Fig. 12]
   Contextual fear conditioning was performed 30 min after intraperitoneal injection of 2-amino-2-thiazoline or saline, and the freezing behavior of mice was measured the next day in the same context, the results of which are shown.
[Fig. 13]
   The structural formulas of the compounds used in Examples 3 to 12 are shown.

### [Description of Embodiments]

The ring A in the formula (I) is a 5- to 7-membered heterocycle containing 1 or 2 hetero atoms selected from a nitrogen atom, an optionally oxidized sulfur atom and an oxygen atom. The ring A is preferably a 5- to 7-membered heterocycle containing 1 or 2 hetero atoms selected from a nitrogen atom and an optionally oxidized sulfur atom. The ring A is more preferably a 5- to 7-membered heterocycle containing a nitrogen atom and an optionally oxidized sulfur atom. The number of members of ring A is more preferably 5 or 6.

Examples of the aforementioned heterocycle includes, but are not limited to, pyrrole, pyridine, pyridazine, pyrimidine, pyrazine, piperazine, pyrrolidine, hexahydropyridazine, imidazole, imidazolidine, piperidine, thiophene, thiolane, tetrahydro-2H-thiopyran, thiazoline (e.g., 2-thiazoline, 3-thiazoline, 4-thiazoline), thiazole, thiazolidine, isothiazole, isothiazoline, thiomorpholine, thiadiazoline, thiadiazole, thiadiazolidine, 1,3-thiazinane, 5,6-dihydro-4H-1,3-thiazine, 2,3-dihydro-4H-1,4-thiazine, furan, 2H-pyran, 4H-pyran, oxazole, isoxazole, morpholine, oxazoline, azepane, and the like. It is preferably thiazoline (e.g., 2-thiazoline, 3-thiazoline, 4-thiazoline), thiazole, thiazolidine, thiomorpholine, thiophene, pyrrole, morpholine, azepane, pyridine, pyrazine, furan, 2,3-dihydro-4H-1,4-thiazine, or imidazole, further preferably thiazoline (e.g., 2-thiazoline, 3-thiazoline), thiazole, thiazolidine, thiomorpholine, thiophene, or 2,3-dihydro-4H-1,4-thiazine. Other preferred examples of ring A include thiazoline (e.g., 2-thiazoline, 3-thiazoline), thiazole, thiomorpholine, pyridine, pyrazine, and pyrrole.

The "halogen atom" used here is preferably selected from a fluorine atom, a chlorine atom, a bromine atom, and an iodine atom.

The "C₁₋₆ alkyl group" used here (when used as a group or a part of a group) is a straight chain or branched chain alkyl group having 1 to 6 carbon atoms. Examples of the C₁₋₆ alkyl group include, but are not limited to, methyl group, ethyl group, propyl group, isopropyl group, butyl group, 1-methylpropyl group (sec-butyl group), 2-methylpropyl group (isobutyl group), tert-butyl group, pentyl group, 1-methylbutyl group, 2-methylbutyl group, 3-methylbutyl group, 1,1-dimethylpropyl group, 2,2-dimethylpropyl group, 1,2-dimethylpropyl group, 1-ethylpropyl group, hexyl group, 1-methylpentyl group, 2-methylpentyl group, 3-methylpentyl group, 4-methylpentyl group, 1,1-dimethylbutyl group, 2,2-dimethylbutyl group, 3,3-dimethylbutyl group, 1,2-dimethylbutyl group, 1,3-dimethylbutyl group, 2,3-dimethylbutyl group, 1-ethylbutyl group, 2-ethylbutyl group, 1-ethyl-2-methylpropyl group, and the like. Preferred examples of the C₁₋₆ alkyl group include C₁₋₄ alkyl group (straight chain or branched chain alkyl group having 1 to 4 carbon atoms). It is further preferably methyl group, ethyl group, propyl group, isopropyl group, butyl group, isobutyl group, or sec-butyl group, and particularly preferably methyl group or ethyl group.

The "C₁₋₆ haloalkyl group" used here means a C₁₋₆ alkyl group substituted by 1 to 5 halogeno groups. When two or more halogeno groups are present, the kind of respective halogeno groups may be the same or different. As the halogeno group, a fluoro group, a chloro group, a bromo group, and the like can be mentioned. Examples of the C₁₋₆ haloalkyl group include, but are not limited to, fluoromethyl group, difluoromethyl group, trifluoromethyl group, chlorodifluoromethyl group, 1-fluoroethyl group, 2-fluoroethyl group, 2-chloroethyl group, 2-bromoethyl group, 1,1-difluoroethyl group, 1,2-difluoroethyl group, 2,2,2-trifluoroethyl group, 1,1,2,2-tetrafluoroethyl group, 1,1,2,2,2-pentafluoroethyl group, 1-fluoropropyl group, 1,1-difluoropropyl group, 2,2-difluoropropyl group, 3-fluoropropyl group, 3,3,3-trifluoropropyl group, 4-fluorobutyl group, 4,4,4-trifluorobutyl group, 5-fluoropentyl group, 5,5,5-trifluoropentyl group, 6-fluorohexyl group, 6,6,6-trifluorohexyl group, and the like.

The "C₂₋₆ alkenyl group" used here (when used as a group or a part of a group) means a straight chain or branched chain alkenyl group having 2 to 6 carbon atoms. Examples of the C₂₋₆ alkenyl group include, but are not limited to, vinyl group, allyl group, prop-1-enyl group, but-1-en-1-yl group, but-2-en-1-yl group, pent-4-en-1-yl group, 2-methylallyl group, and the like.

The "C₁₋₆ alkoxy group" used here (when used as a group or a part of a group) means a straight chain or branched chain alkoxy group having 1 to 6 carbon atoms. Examples of the C₁₋₆ alkoxy group include, but are not limited to, methoxy group, ethoxy group, propoxy group, isopropoxy group, butoxy group, 1-methylpropoxy group, 2-methylpropoxy group, tert-butoxy group, pentyloxy group, 1-methylbutoxy group, 2-methylbutoxy group, 3-methylbutoxy group, 1,1-dimethylpropoxy group, 2,2-dimethylpropoxy group, 1,2-dimethylpropoxy group, 1-ethylpropoxy group, hexyloxy group, and the like.

The "C₁₋₆ alkylthio group" used here means an -SH group substituted by a C₁₋₆ alkyl group. Examples of the C₁₋₆ alkylthio group include, but are not limited to, methylthio group, ethylthio group, propylthio group, butylthio group, and the like.

The "C₂₋₆ alkenylthio group" used here means an -SH group substituted by C₂₋₆ alkenyl. Examples of the C₂₋₆ alkenylthio group include, but are not limited to, vinylthio group, allylthio group, prop-1-enylthio group, but-1-en-1-ylthio group, but-2-en-1-ylthio group, pent-4-en-1-ylthio group, 2-methylallylthio group, and the like.

The "C₁₋₆ alkyl-carbonyl group" used here means a carbonyl group bonded to a C₁₋₆ alkyl group. Examples of the C₁₋₆ alkyl-carbonyl group include, but are not limited to, acetyl group, propionyl group, butyryl group, isobutyryl group, valeryl group, hexanoyl group, and the like.

The "C₁₋₆ alkoxycarbonyl group" used here means a carbonyl group bonded to a C₁₋₆ alkoxy group. Examples of the C₁₋₆ alkoxycarbonyl group include, but are not limited to, methoxycarbonyl group, ethoxycarbonyl group, propoxycarbonyl group, isopropoxycarbonyl group, butoxycarbonyl group, and the like.

The "C₆₋₁₀ aryl group" used here means an aromatic hydrocarbon group having 6 to 10 carbon atoms. Examples of the C₆₋₁₀ aryl group include, but are not limited to, phenyl group, naphthyl group (1-naphthyl group, 2-naphthyl group), and the like.

The "5- or 6-membered heteroaryl group" used here means a 5- or 6-membered heteroaryl group containing at least 1 (preferably 1 to 3, more preferably 1 or 2) hetero atom selected from nitrogen atom, optionally oxidized sulfur atom and oxygen atom. The 5- or 6-membered heteroaryl group is preferably a 5- or 6-membered heteroaryl group containing 1 or 2 hetero atoms selected from nitrogen atom and optionally oxidized sulfur atom.

Examples of the 5- or 6-membered heteroaryl group include, but are not limited to, pyrrolyl group, pyridyl group, pyridazinyl group, pyrimidinyl group, pyrazinyl group, imidazolyl group, thienyl group, thiazolyl group, isothiazolyl group, thiadiazolyl group, furyl group, oxazolyl group, isoxazolyl group, and the like. Preferably, it is pyridyl group, thienyl group, and the like.

The term "oxo group" used here (when used as a group or a part of a group) shows an =O group.

The term "optionally oxidized sulfur atom" used here means S, SO, or SO₂.

The "5- or 6-membered ring" of the "optionally substituted 5- or 6-membered ring", which is formed by R¹ and R² bonded to each other, means a 5- or 6-membered ring optionally having at least 1 (preferably 1 to 3, more preferably 1 or 2) hetero atom selected from a nitrogen atom, an optionally oxidized sulfur atom and an oxygen atom. Examples of the aforementioned 5- or 6-membered ring include benzene ring, tetrahydropyrimidine ring, and the like. The aforementioned 5- or 6-membered ring may be substituted, and examples of the substituent include 1 to 4 (preferably 1 or 2) substituents selected from C₁₋₆ alkyl group, C₁₋₆ alkoxy group, halogen atom, amino group, -SH, C₁₋₆ alkylthio group, C₂₋₆ alkenylthio group, C₁₋₆ alkyl-carbonyl group, formyl group, C₁₋₆ alkoxycarbonyl group, oxo group, and the like. The substituent is preferably 1 to 4 substituents selected from C₁₋₆ alkyl group (e.g., methyl) and oxo group.

In the formula (I), preferably, R¹, R², R³ and R⁴ are each independently hydrogen atom, C₁₋₆ alkyl group (e.g., methyl, ethyl, propyl, isopropyl, butyl, isobutyl, sec-butyl, tert-butyl), C₁₋₆ alkoxy group (e.g., methoxy, ethoxy), halogen atom (e.g., chlorine atom), amino group, -SH, C₁₋₆ alkylthio group (e.g., methylthio), C₂₋₆ alkenylthio group (e.g., allylthio), C₁₋₆ alkyl-carbonyl group (e.g., acetyl), formyl group, C₆₋₁₀ aryl group (e.g., phenyl), 5- or 6-membered heteroaryl group (e.g., thienyl), or oxo group; and R¹ and R² are optionally bonded to each other to form an optionally substituted 5- or 6-membered ring (e.g., benzene ring, tetrahydropyrimidine ring).

In the formula (I), when n=1 or 2, it is preferred that at least one selected from R¹, R², R³ and R⁴ is not a hydrogen atom. In the formula (I), when n=0, it is preferred that at least one selected from R¹, R² and R³ is not a hydrogen atom.

Examples of the preferred heterocyclic compound of the formula (I) used as the active ingredient in the present invention include, but are not limited to, 2-methyl-2-thiazoline (2MT), 5-methylthiazole, 4-ethyl-2-methyl-2-thiazoline (4E2MT), 2,4,5-trimethyl-3-thiazoline, thiomorpholine, 2,3-diethylpyrazine, 2,6-lutidine, 2-(allylthio)-2-thiazoline, 4-tert-butyl-2-methylthiazole, 2-ethoxythiazole, 2-methylbenzothiazole, 2-mercaptothiazole, 2-methylthiomorpholine, 2-acetylpyrrole, 2-methylthiazole, and 2-amino-2-thiazoline.

Examples of the preferred isothiocyanate compound of the formula (II) used as the active ingredient in the present invention include, but are not limited to, allyl isothiocyanate (AITC) and methallyl isothiocyanate.

In the present invention, a heterocyclic compound of the formula (I) and an isothiocyanate compound of the formula (II) used as the active ingredients include substances generally known as reagents, commercially available ones can be utilized, and they can be obtained by a method known per se. Use of the heterocyclic compound of the formula (I) and the isothiocyanate compound of the formula (II) as therapeutic drugs for psychiatric disorders has not been disclosed or suggested to date.

Preferred examples of the heterocyclic compound represented by the formula (I) include compounds represented by the following formulas (A) to (E) or salts thereof. 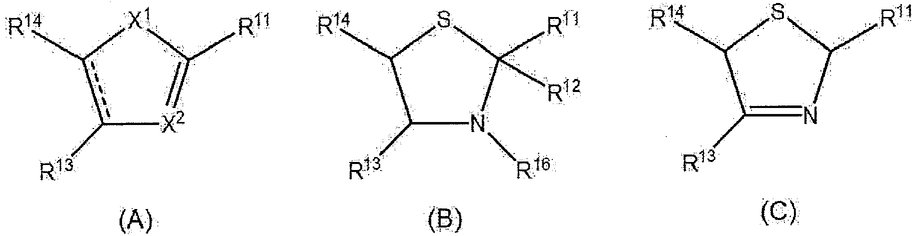 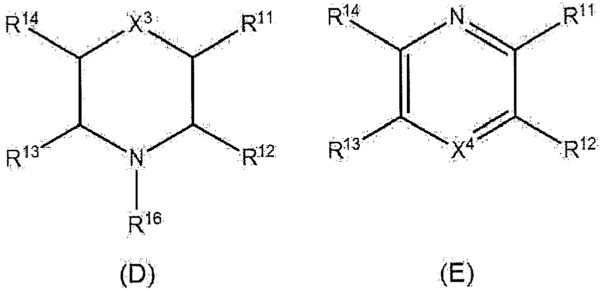 wherein
X¹ is S, O, or N(R¹⁶);
X² is N or CR¹²;
X³ is S, SO₂, O, or -(CH₂)₂-;
X⁴ is N or CR¹⁵;
   - - - - - -
   is a single bond or a double bond;
R¹¹, R¹², R¹³, R¹⁴, R¹⁵, and R¹⁶ are each independently a hydrogen atom, a C₁₋₆ alkyl group, a C₁₋₆ alkoxy group, a halogen atom, an amino group, -SH, a C₁₋₆ alkylthio group, a C₂₋₆ alkenylthio group, a C₁₋₆ alkyl-carbonyl group, a formyl group, a C₆₋₁₀ aryl group, a C₁₋₆ alkoxycarbonyl group, a 5- or 6-membered heteroaryl group, or an oxo group;
R¹³ and R¹⁴ are optionally bonded to each other to form a benzene ring or a tetrahydropyrimidine ring optionally substituted by 1 to 4 substituents selected from a C₁₋₆ alkyl group and an oxo group;
provided that, in the formula (A), R¹¹ and R¹² are not oxo groups; in the formula (A), when
   - - - - - -
   is a double bond, R¹³ and R¹⁴ are not oxo groups;
in the formula (C), R¹³ is not an oxo group;
in the formula (E), R¹¹, R¹², R¹³, R¹⁴ and R¹⁵ are not oxo groups, and
in the formula (B), R¹¹ and R¹² may together form an oxo group.

In the formulas (A) to (E), preferably, R¹¹, R¹², R¹³, R¹⁴, R¹⁵, and R¹⁶ are each independently a hydrogen atom, a C₁₋₆ alkyl group (e.g., methyl, ethyl, propyl, isopropyl, butyl, isobutyl, sec-butyl, tert-butyl), a C₁₋₆ alkoxy group (e.g., methoxy, ethoxy), a halogen atom (e.g., chlorine atom), an amino group, -SH, a C₁₋₆ alkylthio group (e.g., methylthio), a C₂₋₆ alkenylthio group (e.g., allylthio), a C₁₋₆ alkyl-carbonyl group (e.g., acetyl), a formyl group, a C₆₋₁₀ aryl group (e.g., phenyl), a 5- or 6-membered heteroaryl group (e.g., thienyl), or an oxo group;
R¹³ and R¹⁴ are optionally bonded to each other to form a benzene ring or a tetrahydropyrimidine ring optionally substituted by 1 to 4 substituents selected from a C₁₋₆ alkyl group and an oxo group;
provided that, in the formula (A), R¹¹ and R¹² are not oxo groups; in the formula (A), when
   - - - - - -
   is a double bond, R¹³ and R¹⁴ are not oxo groups;
in the formula (C), R¹³ is not an oxo group;
in the formula (E), R¹¹, R¹², R¹³, R¹⁴ and R¹⁵ are not oxo groups, and
in the formula (B), R¹¹ and R¹² may together form an oxo group.

Preferred examples of the heterocyclic compound represented by the formula (I) include compounds represented by the following formulas (A-1), (A-2), (C-1), (D-1), and (E-1), and salts thereof. 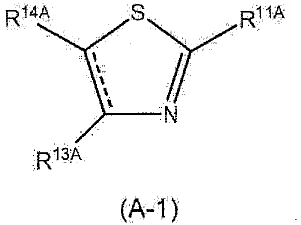 wherein R^{11A} is a hydrogen atom, a C₁₋₆ alkyl group, a C₁₋₆ alkoxy group, an amino group, -SH, or a C₁₋₆ alkenylthio group; R^{13A} is a hydrogen atom or a C₁₋₆ alkyl group; R^{14A} is a hydrogen atom or a C₁₋₆ alkyl group; R^{13A} and R^{14A} are optionally bonded to each other to form a benzene ring; and
is a single bond or a double bond.

In a preferred embodiment of the formula (A-1), R^{11A} is a C₁₋₆ alkyl group, a C₁₋₆ alkoxy group, an amino group, -SH, or a C₁₋₆ alkenylthio group; R^{13A} is a hydrogen atom or a C₁₋₆ alkyl group; R^{14A} is a hydrogen atom or a C₁₋₆ alkyl group; and R^{13A} and R^{14A} are optionally bonded to each other to form a benzene ring. In another preferred embodiment of the formula (A-1), R^{11A} is a hydrogen atom or a C₁₋₆ alkyl group; R^{13A} is a hydrogen atom or a C₁₋₆ alkyl group; and R^{14A} is a hydrogen atom or a C₁₋₆ alkyl group.

Preferably, in the formula (A-1), R^{11A} is a hydrogen atom or a C₁₋₄ alkyl group; R^{13A} is a hydrogen atom or a C₁₋₄ alkyl group; and R^{14A} is a hydrogen atom or a C₁₋₄ alkyl group.

In the formula (A-1), at least one selected from R^{11A}, R^{13A}, and R^{14A} is more preferably not a hydrogen atom.

Further preferably, in the formula (A-1), R^{11A} is a C₁₋₄ alkyl group; R^{13A} is a hydrogen atom or a C₁₋₄ alkyl group; R^{14A} is a hydrogen atom; and

is a single bond. 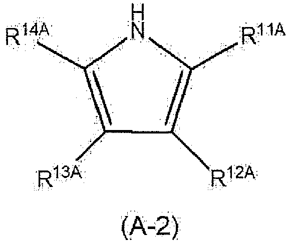 wherein R^{11A} is a hydrogen atom, a C₁₋₆ alkyl group, or a C₁₋₆ alkyl-carbonyl group; R^{12A} is a hydrogen atom or a C₁₋₆ alkyl group; R^{13A} is a hydrogen atom or a C₁₋₆ alkyl group; and R^{14A} is a hydrogen atom or a C₁₋₆ alkyl group.

In the formula (A-2), at least one selected from R^{11A}, R^{12A}, R^{13A}, and R^{14A} is preferably not a hydrogen atom.

Further preferably, in the formula (A-2), R^{11A} is a C₁₋₆ alkyl-carbonyl group; and R^{12A}, R^{13A}, and R^{14A} are each a hydrogen atom. 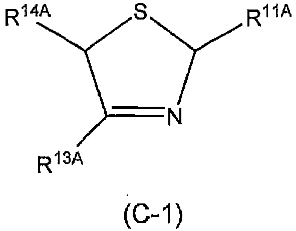 wherein R^{11A} is a hydrogen atom or a C₁₋₆ alkyl group; R^{13A} is a hydrogen atom or a C₁₋₆ alkyl group; and R^{14A} is a hydrogen atom or a C₁₋₆ alkyl group.

Preferably, in the formula (C-1), R^{11A} is a hydrogen atom or a C₁₋₄ alkyl group; R^{13A} is a hydrogen atom or a C₁₋₄ alkyl group; and R^{14A} is a hydrogen atom or a C₁₋₄ alkyl group.

In the formula (C-1), at least one selected from R^{11A}, R^{13A}, and R^{14A} is preferably not a hydrogen atom.

Further preferably, in the formula (C-1), R^{11A} is a C₁₋₄ alkyl group; R^{13A} is a C₁₋₄ alkyl group; and R^{14A} is a C₁₋₄ alkyl group. 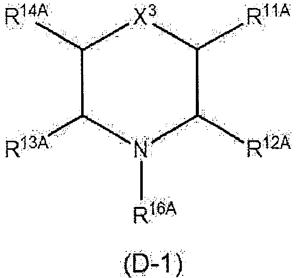 wherein X³ is S or SO₂; R^{11A} is a hydrogen atom or a C₁₋₆ alkyl group; R^{12A} is a hydrogen atom or a C₁₋₆ alkyl group; R^{13A} is a hydrogen atom or a C₁₋₆ alkyl group; R^{14A} is a hydrogen atom or a C₁₋₆ alkyl group; and R^{16A} is a hydrogen atom or a C₁₋₆ alkyl group.

Preferably, in the formula (D-1), R^{11A} is a hydrogen atom or a C₁₋₄ alkyl group; R^{12A} is a hydrogen atom or a C₁₋₄ alkyl group; R^{13A} is a hydrogen atom or a C₁₋₄ alkyl group; R^{14A} is a hydrogen atom or a C₁₋₄ alkyl group; and R^{16A} is a hydrogen atom or a C₁₋₄ alkyl group.

Further preferably, in the formula (D-1), X³ is S; R^{11A} is a hydrogen atom or a C₁₋₄ alkyl group; and R^{12A}, R^{13A}, R^{14A}, and R^{16A} are each a hydrogen atom. 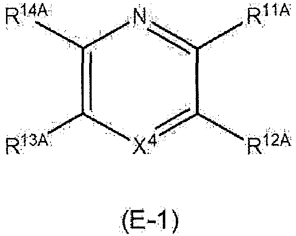 wherein X⁴ is N or CR^{15A}; R^{11A} is a hydrogen atom or a C₁₋₆ alkyl group; R^{12A} is a hydrogen atom or a C₁₋₆ alkyl group; R^{13A} is a hydrogen atom or a C₁₋₆ alkyl group; R^{14A} is a hydrogen atom or a C₁₋₆ alkyl group; and R^{15A} is a hydrogen atom or a C₁₋₆ alkyl group.

Preferably, in the formula (E-1), R^{11A} is a hydrogen atom or a C₁₋₄ alkyl group; R^{12A} is a hydrogen atom or a C₁₋₄ alkyl group; R^{13A} is a hydrogen atom or a C₁₋₄ alkyl group; R^{14A} is a hydrogen atom or a C₁₋₄ alkyl group; and R^{15A} is a hydrogen atom or a C₁₋₄ alkyl group.

In the formula (E-1), at least one selected from R^{11A}, R^{12A}, R^{13A}, R^{14A}, and R^{15A} is more preferably not a hydrogen atom.

In a preferred embodiment of the formula (E-1), R^{11A} is a C₁₋₄ alkyl group; R^{14A} is a C₁₋₄ alkyl group; and R^{12A}, R^{13A}, and R^{15A} are each a hydrogen atom.

In another preferred embodiment of the formula (E-1), R^{11A} is a C₁₋₄ alkyl group; R^{12A} is a C₁₋₄ alkyl group; and R^{13A}, R^{14A}, and R^{15A} are each a hydrogen atom.

A preferred example of the isothiocyanate compound represented by the formula (II) is an isothiocyanate compound wherein R⁵ is a C₁₋₆ alkyl group or a C₂₋₆ alkenyl group, more preferably an isothiocyanate compound wherein R⁵ is a C₂₋₆ alkenyl group.

The salt of the compound of the present invention may be a pharmaceutically acceptable salt. For example, alkali metal salts such as sodium salt and potassium salt; alkaline earth metal salts such as magnesium salt and calcium salt; ammonium salts such as dimethylammonium salt and triethylammonium salt; inorganic acid salts such as hydrochloride, perchlorate, sulfate and nitrate; organic acid salts such as acetate and methanesulfonate; and the like can be mentioned.

A prophylactic or therapeutic agent for psychiatric disorders, which is provided by the present invention, can be utilized as a prophylactic or therapeutic agent for psychiatric disorders.

Examples of the psychiatric disorder include post traumatic stress disorder, depression, panic disorder, anxiety disorder, dissociative disorder, obsessive-compulsive disorder, eating disorder, sleep disorder, bipolar disorder, adjustment disorder, schizophrenia, epilepsy, and the like.

A heterocyclic compound of the formula (I) or a salt thereof, or an isothiocyanate compound of the formula (II) (hereinafter also to be referred to as the compound of the present invention) can be administered to animals, including a human who has or may develop a psychiatric disorder, for the purpose of preventing the development of disorder or alleviating the symptoms. A gas derived from the compound of the present invention and developed at a concentration of 0.1 to 100,000 ppm can be inhaled through the nasal cavity or lung using a gas mask or a device having a similar function. Alternatively, the compound of the present invention can be administered orally at a dose of 1 µg/kg to 5,000 mg/kg. Alternatively, the compound of the present invention can be intracorporeally injected at a dose of 1 µg/kg to 5,000 mg/kg by a method such as intradermal injection, subcutaneous injection, intramuscular injection, intravenous injection, intraarterial injection, intraspinal injection, intraperitoneal injection, and the like. The administration frequency may be single-dose administration, or continuous administration at regular intervals, or continuous administration at different time intervals. The animal to be the subject of administration may be, for example, mammals (human, mouse, rat, hamster, rabbit, cat, dog, bovine, sheep, swine, horse, monkey, and the like).

When the compound of the present invention is used as a prophylactic or therapeutic agent for psychiatric disorders (hereinafter to be also referred to as the agent of the present invention), a pharmaceutically acceptable additive can be added as necessary.

Specific examples of the pharmaceutically acceptable additive include, but are not limited to, antioxidant, preservative, colorant, flavoring agent, and diluent, emulsifier, suspending agent, solvent, filler, extending agent, buffering agent, delivery vehicle, diluent, carrier, excipient and/or pharmaceutical adjuvant, and the like.

The dosage form of the agent of the present invention is not particularly limited and, for example, liquid, injection, sustained-release preparation, and the like can be mentioned. The solvent to be used for formulating the agent of the present invention in the above-mentioned dosage form may be aqueous or non-aqueous.

An injection can be prepared by a method well known in the pertinent field. For example, an injection can be prepared by dissolving in an appropriate solvent (saline, buffer such as PBS, sterile water, and the like), sterilizing by filtration with a filter or the like, and then filling in an aseptic container (e.g., ampoule and the like). The injection may contain a conventionally-used pharmacological carrier as necessary. An administration method using a non-invasive catheter can also be adopted. The carrier that can be used in the present invention includes neutral buffered saline, saline containing serum albumin, and the like.

### [Examples]

The present invention is explained in more detail in the following by referring to Examples. The Examples do not limit the present invention.

### Example 1: Inhibition of fear memory formation by thiazoline-related fear odor

### Experiment method

Day 0: About 2-month-old C57/BL6 mice were placed in an electric shock chamber, and 2-second electric shock (0.6 mA) was given 6 times at 2-minute intervals for 12 min for contextual fear conditioning. Conditioning was performed under 4 conditions of no odor condition (control; n=6) (group 1) and an electric shock chamber with filter paper impregnated with 100 µl of 2-methyl-2-thiazoline (2MT) condition (2MT; n=6) (group 2), or no odor condition (control; n=6) (group 3) and an electric shock chamber with filter paper impregnated with 10 µl of 2-methyl-2-thiazoline (2MT) condition (2MT; n=6) (group 4). Day 1: Mice were placed in the electric shock chamber (no odor) used for learning on the previous day, and freezing behavior was measured for 10 min using dedicated software (Freeze frame 2; ActiMetrics).

Day 3: The mice of group 1 and group 2 were placed in an electric shock chamber with filter paper impregnated with 100 µl of Eugenol, and freezing behavior was measured for 10 min. The mice in group 3 and group 4 were placed in an electric shock chamber with filter paper impregnated with 10 µl of Eugenol, and freezing behavior was measured for 10 min.

### Results

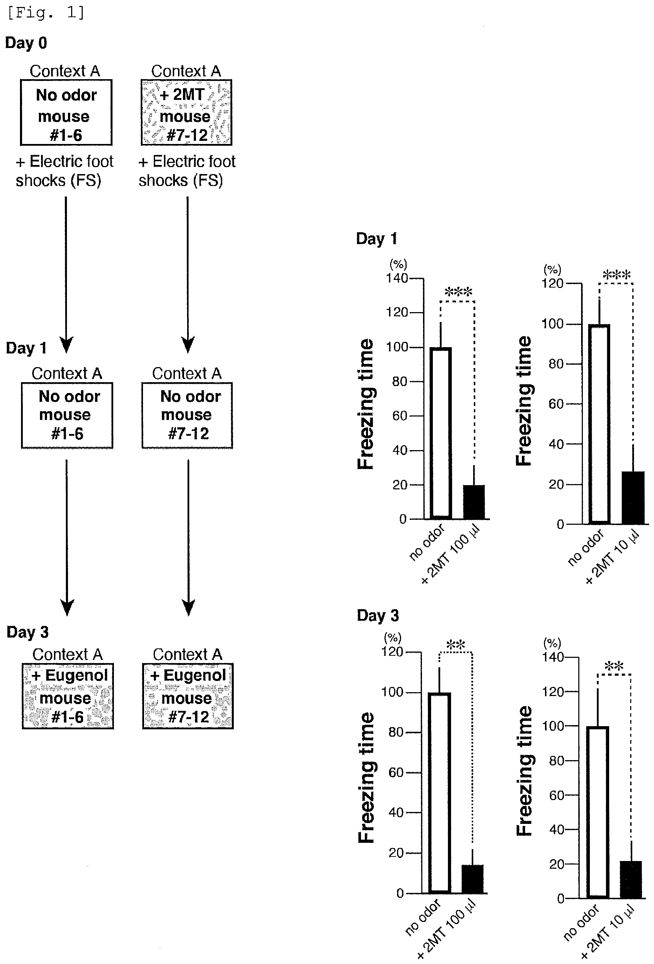

The results are shown in Fig. 1.

The left Figure shows the timeline of the experiment, and the right Figure shows the frequency of freezing behavior (mean ± standard error) for 10 min in the electric shock chamber on day 1 and day 3, in which the group with no odor is indicated by a white bar graph and the group with 2MT is indicated by a black bar graph. A student's t-test was also performed between the group with no odor and the group with 2MT (** p<0.01, *** p<0.001).

In the group exposed to 2MT during fear conditioning, the freezing behavior in the no odor context (day 1) remarkably decreased compared to that in the no odor group. This result suggests two possibilities: 2MT may have inhibited the formation of fear memory, and the group that learned in the presence of 2MT recognized that the context during the test is different from the context during learning due to the absence of odor in the test environment. However, the group exposed to 2MT during fear conditioning showed a marked decrease in the freezing behavior even in the context with odor (eugenol odor context; day 3) compared to the group that learned with no odor. Therefore, it is unlikely that the conditions of presence or absence of odor affected the fear learning, and it is highly likely that 2MT inhibited the formation of fear memory itself. The above results suggest that 2MT has a strong inhibitory effect on fear memory formation.

### Example 2: Inhibition of fear memory formation by intraperitoneal administration of thiazoline-related fear odors Experiment Method

Day 0: To about 2 month-old C57/BL6 mice was intraperitoneally administered 200 µl of saline, or 1 v/v% 2-methyl-2-thiazoline solution (dissolved in saline), or 1 v/v% 5-methylthiazole solution (dissolved in saline), or 1 v/v% 4-ethyl-2-methyl-2-thiazoline solution (dissolved in saline), or 1 v/v% 2,4,5-trimethyl-3-thiazoline solution (dissolved in saline), or 1 v/v% thiomorpholine solution (dissolved in saline), or 1 v/v% allyl isothiocyanate (AITC) solution (dissolved in saline), or 1 v/v% 2,3-diethylpyrazine solution (dissolved in saline), or 1 v/v% 2,6-lutidine solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

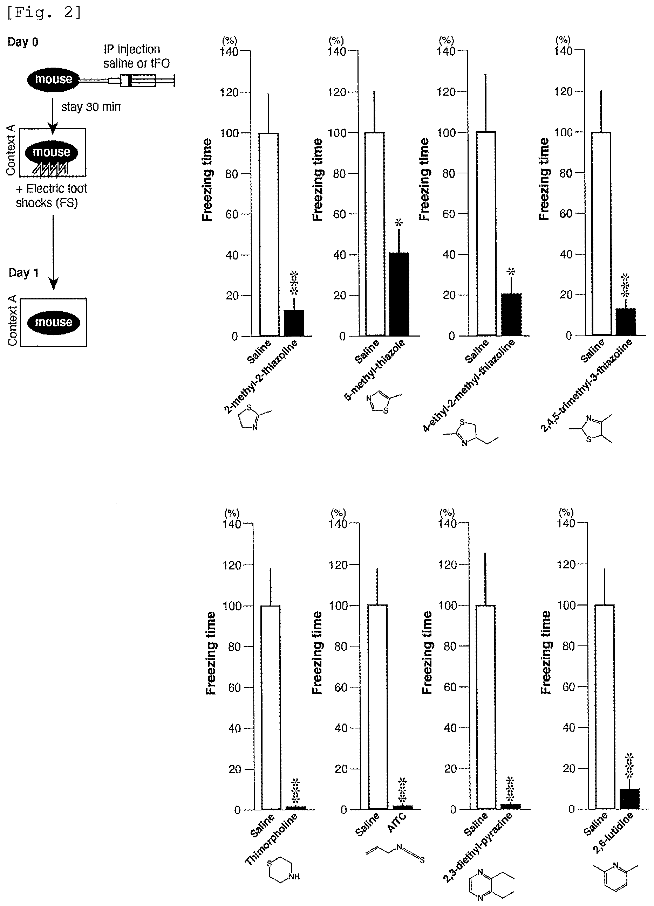

The results are shown in Fig. 2.

The left Figure shows the timeline of the experiment, and the right Figure shows the frequency of freezing behavior (mean ± standard error) for 10 min in the electric shock chamber during the test (day 1), in which the mean frequency of the freezing behavior when saline was administered was 100%. In addition, a student's t-test was performed between the saline-administered group and each compound-administered group (* p<0.05, *** p<0.001).

The group administered with 2-methyl-2-thiazoline, 5-methylthiazole, 4-ethyl-2-methyl-2-thiazoline, 2,4,5-trimethyl-3-thiazoline, thiomorpholine, AITC, 2,3-diethylpyrazine, or 2,6-lutidine showed a significant decrease in the frequency of freezing behaviors as compared with the saline-administered group. Thus, it was suggested that 2-methyl-2-thiazoline, 5-methylthiazole, 4-ethyl-2-methyl-2-thiazoline, 2,4,5-trimethyl-3-thiazoline, thiomorpholine, AITC, 2,3-diethylpyrazine, and 2,6-lutidine have a strong inhibitory effect on fear memory formation.

### Example 3: Inhibition of fear memory formation by intraperitoneal administration of 2-(allylthio)-2-thiazoline Experiment Method

Day 0: To about 2 month-old C57/BL6 mice was intraperitoneally administered 200 µl of saline or 1 v/v% 2-(allylthio)-2-thiazoline solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

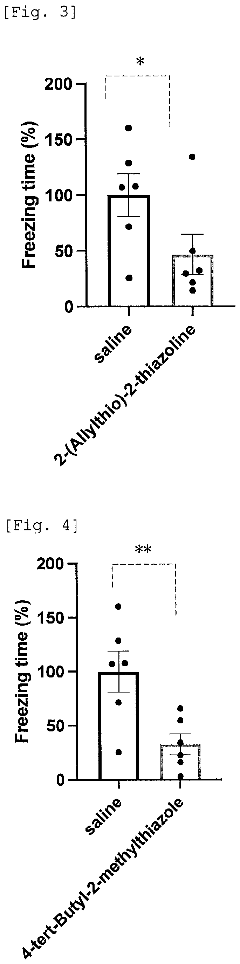

The results are shown in Fig. 3.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 2-(allylthio)-2-thiazoline-administered group (* p<0.05).

### Example 4: Inhibition of fear memory formation by intraperitoneal administration of 4-tert-butyl-2-methylthiazole Experiment Method

Day 0: To about 2 month-old C57/BL6 mice was intraperitoneally administered 200 µl of saline or 1 v/v% 4-tert-butyl-2-methylthiazole solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

The results are shown in Fig. 4.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 4-tert-butyl-2-methylthiazole-administered group (** p<0.01).

### Example 5: Inhibition of fear memory formation by intraperitoneal administration of 2-ethoxythiazole Experiment Method

Day 0: To about 2 month-old C57/BL6 mice was intraperitoneally administered 200 µl of saline or 1 v/v% 2-ethoxythiazole solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

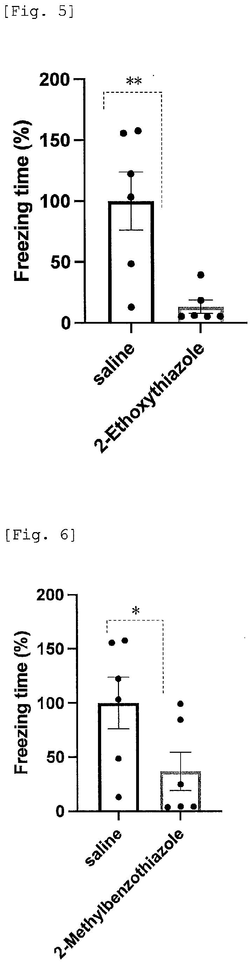

The results are shown in Fig. 5.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 2-ethoxythiazole-administered group (** p<0.01) .

### Example 6: Inhibition of fear memory formation by intraperitoneal administration of 2-methylbenzothiazole Experiment Method

Day 0: To about 2 month-old C57/BL6 mice was intraperitoneally administered 200 µl of saline or 1 v/v% 2-methylbenzothiazole solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

The results are shown in Fig. 6.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 2-methylbenzothiazole-administered group (* p<0.05).

### Example 7: Inhibition of fear memory formation by intraperitoneal administration of 2-mercaptothiazole Experiment Method

Day 0: To about 2 month-old C57/BL6 mice was intraperitoneally administered 200 µl of saline or 1 v/v% 2-mercaptothiazole solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

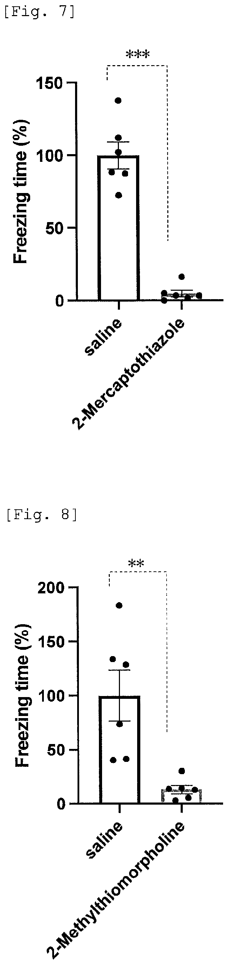

The results are shown in Fig. 7.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 2-mercaptothiazole-administered group (*** p<0.001).

### Example 8: Inhibition of fear memory formation by intraperitoneal administration of 2-methylthiomorpholine Experiment Method

Day 0: To about 2 month-old C57/BL6 mice was intraperitoneally administered 200 µl of saline or 1 v/v% 2-methylthiomorpholine solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

The results are shown in Fig. 8.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 2-methylthiomorpholine-administered group (** p<0.01).

### Example 9: Inhibition of fear memory formation by intraperitoneal administration of 2-acetylpyrrole Experiment Method

Day 0: To about 2 month-old C57/BL6 mice was intraperitoneally administered 200 µl of saline or 1 v/v% 2-acetylpyrrole solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

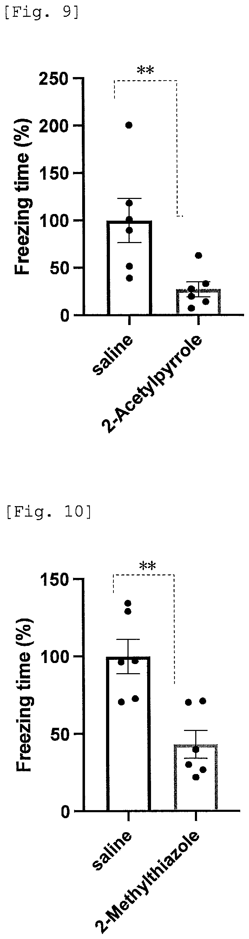

The results are shown in Fig. 9.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 2-acetylpyrrole-administered group (** p<0.01).

### Example 10: Inhibition of fear memory formation by intraperitoneal administration of 2-methylthiazole Experiment Method

Day 0: To about 2 month-old C57/BL6 mice was intraperitoneally administered 200 µl of saline or 1 v/v% 2-methylthiazole solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

The results are shown in Fig. 10.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 2-methylthiazole-administered group (** p<0.01).

### Example 11: Inhibition of fear memory formation by intraperitoneal administration of methallyl isothiocyanate Experiment Method

Day 0: To about 2 month-old C57/BL6 mice was intraperitoneally administered 200 µl of saline or 1 v/v% methallyl isothiocyanate solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

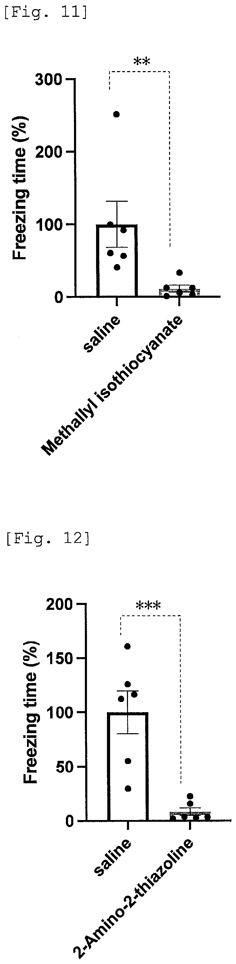

The results are shown in Fig. 11.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the methallyl isothiocyanate-administered group (** p<0.01) .

### Example 12: Inhibition of fear memory formation by intraperitoneal administration of 2-amino-2-thiazoline Experiment Method

Day 0: To about 2 month-old C57/BL6 mice was intraperitoneally administered 200 µl of saline or 1 v/v% 2-amino-2-thiazoline solution (dissolved in saline) (each condition n=6). Thirty minutes after the administration, the mice were placed in an electric shock chamber, and 2-second electric shock (0.6 mA) was given 6 times at 2-minute intervals for 12 min for contextual fear conditioning.

Day 1: Mice were placed in the electric shock chamber (no odor) used for learning on the previous day, and freezing behavior was measured for 10 min using dedicated software (Freeze frame 2) .

### Results

The results are shown in Fig. 12.

The Figure shows the frequency of freezing behavior (mean ± standard error) for 10 min in the electric shock chamber during the test (day 1), in which the mean frequency of the freezing behavior when saline was administered was 100%. A student's t-test was performed between the saline-administered group and the 2-amino-2-thiazoline-administered group (** *p<0.001).

The group administered with 2-(allylthio)-2-thiazoline, 4-tert-butyl-2-methylthiazole, 2-ethoxythiazole, 2-methylbenzothiazole, 2-mercaptothiazole, 2-methylthiomorpholine, 2-acetylpyrrole, 2-methylthiazole, methallyl isothiocyanate, or 2-amino-2-thiazoline showed a significant decrease in the freezing behaviors as compared with the saline-administered group. Thus, it was suggested that 2-(allylthio)-2-thiazoline, 4-tert-butyl-2-methylthiazole, 2-ethoxythiazole, 2-methylbenzothiazole, 2-mercaptothiazole, 2-methylthiomorpholine, 2-acetylpyrrole, 2-methylthiazole, methallyl isothiocyanate, and 2-amino-2-thiazoline have a strong inhibitory effect on fear memory formation.

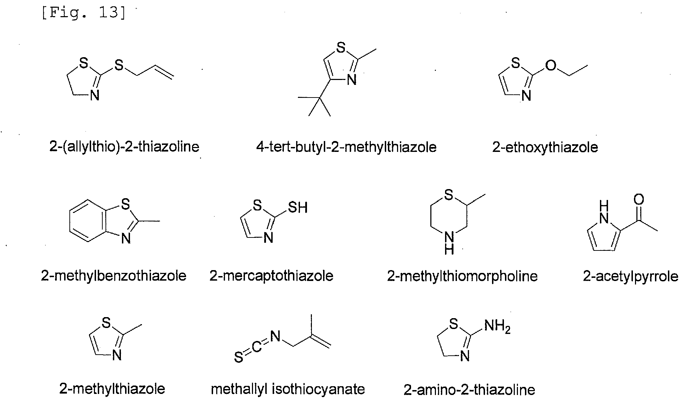

The structural formulas of the compounds used in Examples 3 to 12 are shown in Fig. 13.

### [Industrial Applicability]

The prophylactic or therapeutic agent for psychiatric disorders of the present invention can induce psychotropic actions according to the principle of inducing latent life-protective abilities by selectively activating the sensory nerves, and can be used for the prophylaxis or treatment of psychiatric disorders.

This application is based on patent application No. 2020-131872 filed in Japan, the contents of which are incorporated by reference in full herein.

## Claims

1. A prophylactic or therapeutic agent for a psychiatric disorder, comprising, as an active ingredient, at least one kind selected from a heterocyclic compound represented by the formula (I) 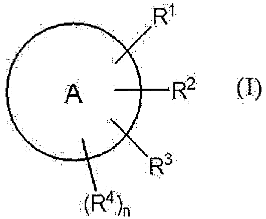 wherein
ring A is a 5- to 7-membered heterocycle containing 1 or 2 hetero atoms selected from a nitrogen atom, an optionally oxidized sulfur atom and an oxygen atom;
R¹, R², R³ and R⁴ are each independently a hydrogen atom, a C₁₋₆ alkyl group, a C₁₋₆ alkoxy group, a halogen atom, an amino group, -SH, a C₁₋₆ alkylthio group, a C₂₋₆ alkenylthio group, a C₁₋₆ alkyl-carbonyl group, a formyl group, a C₆₋₁₀ aryl group, a C₁₋₆ alkoxycarbonyl group, a 5- or 6-membered heteroaryl group, or an oxo group; R¹ and R² are optionally bonded to each other to form an optionally substituted 5- or 6-membered ring; and n is 0, 1, or 2
or a salt thereof, and
an isothiocyanate compound represented by the formula (II)
S=C=N-R⁵ (II)
wherein R⁵ is a C₁₋₆ alkyl group, a C₁₋₆ haloalkyl group, a C₂₋₆ alkenyl group, a C₆₋₁₀ aryl group, or a 5- or 6-membered heteroaryl group.

2. The agent according to claim 1, wherein the ring A is thiazoline, thiazole, thiazolidine, thiomorpholine, thiophene, pyrrole, morpholine, azepane, pyridine, pyrazine, furan, 2,3-dihydro-4H-1,4-thiazine, or imidazole.

3. The agent according to claim 1 or 2, wherein the active ingredient is a heterocyclic compound represented by the formula (I) or a salt thereof.

4. The agent according to claim 1, wherein the active ingredient is an isothiocyanate compound represented by the formula (II).

5. The agent according to any one of claims 1 to 4, wherein the psychiatric disorder is selected from a post traumatic stress disorder, depression, a panic disorder, an anxiety disorder, a dissociative disorder, an obsessive-compulsive disorder, an eating disorder, a sleep disorder, a bipolar disorder, an adjustment disorder, schizophrenia, and epilepsy.

6. The agent according to any one of claims 1 to 5, which is for intranasal administration.

7. Use of at least one kind of compound selected from a heterocyclic compound represented by the formula (I) 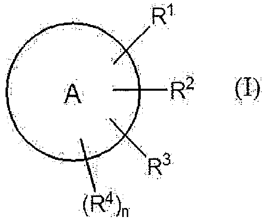 wherein
ring A is a 5- to 7-membered heterocycle containing 1 or 2 hetero atoms selected from a nitrogen atom, an optionally oxidized sulfur atom and an oxygen atom;
R¹, R², R³ and R⁴ are each independently a hydrogen atom, a C₁₋₆ alkyl group, a C₁₋₆ alkoxy group, a halogen atom, an amino group, -SH, a C₁₋₆ alkylthio group, a C₂₋₆ alkenylthio group, a C₁₋₆ alkyl-carbonyl group, a formyl group, a C₆₋₁₀ aryl group, a C₁₋₆ alkoxycarbonyl group, a 5- or 6-membered heteroaryl group, or an oxo group; R¹ and R² are optionally bonded to each other to form an optionally substituted 5- or 6-membered ring; and n is 0, 1, or 2
or a salt thereof, and
an isothiocyanate compound represented by the formula (II)
S=C=N-R⁵ (II)
wherein R⁵ is a C₁₋₆ alkyl group, a C₁₋₆ haloalkyl group, a C₂₋₆ alkenyl group, a C₆₋₁₀ aryl group, or a 5- or 6-membered heteroaryl group,
for the production of a prophylactic or therapeutic agent for a psychiatric disorder.

8. The use according to claim 7, wherein the ring A is thiazoline, thiazole, thiazolidine, thiomorpholine, thiophene, pyrrole, morpholine, azepane, pyridine, pyrazine, furan, 2,3-dihydro-4H-1,4-thiazine, or imidazole.

9. The use according to claim 7 or 8, wherein the compound is a heterocyclic compound represented by the formula (I) or a salt thereof.

10. The use according to claim 7, wherein the compound is an isothiocyanate compound represented by the formula (II).

11. The use according to any one of claims 7 to 10, wherein the psychiatric disorder is selected from a post traumatic stress disorder, depression, a panic disorder, an anxiety disorder, a dissociative disorder, an obsessive-compulsive disorder,an eating disorder, a sleep disorder, a bipolar disorder, an adjustment disorder, schizophrenia, and epilepsy.

12. The use according to any one of claims 7 to 11, wherein the prophylactic or therapeutic agent is for intranasal administration.

13. A method for preventing or treating a psychiatric disorder in a mammal, comprising administering to the mammal an effective amount of at least one kind of compound selected from a heterocyclic compound represented by the formula (I) 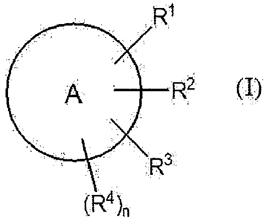 wherein
ring A is a 5- to 7-membered heterocycle containing 1 or 2 hetero atoms selected from a nitrogen atom, an optionally oxidized sulfur atom and an oxygen atom;
R¹, R², R³ and R⁴ are each independently a hydrogen atom, a C₁₋₆ alkyl group, a C₁₋₆ alkoxy group, a halogen atom, an amino group, -SH, a C₁₋₆ alkylthio group, a C₂₋₆ alkenylthio group, a C₁₋₆ alkyl-carbonyl group, a formyl group, a C₆₋₁₀ aryl group, a C₁₋₆ alkoxycarbonyl group, a 5- or 6-membered heteroaryl group, or an oxo group; R¹ and R² are optionally bonded to each other to form an optionally substituted 5- or 6-membered ring; and n is 0, 1, or 2
or a salt thereof, and
an isothiocyanate compound represented by the formula (II)
S=C=N-R⁵ (II)
wherein R⁵ is a C₁₋₆ alkyl group, a C₁₋₆ haloalkyl group, a C₂₋₆ alkenyl group, a C₆₋₁₀ aryl group, or a 5- or 6-membered heteroaryl group.

14. The method according to claim 13, wherein the ring A is thiazoline, thiazole, thiazolidine, thiomorpholine, thiophene, pyrrole, morpholine, azepane, pyridine, pyrazine, furan, 2,3-dihydro-4H-1,4-thiazine, or imidazole.

15. The method according to claim 13 or 14, wherein the compound is a heterocyclic compound represented by the formula (I) or a salt thereof.

16. The method according to claim 13, wherein the compound is an isothiocyanate compound represented by the formula (II).

17. The method according to any one of claims 13 to 16, wherein the psychiatric disorder is selected from a post traumatic stress disorder, depression, a panic disorder, an anxiety disorder, a dissociative disorder, an obsessive-compulsive disorder, an eating disorder, a sleep disorder, a bipolar disorder, an adjustment disorder, schizophrenia, and epilepsy.

18. The method according to any one of claims 13 to 17, wherein the compound is intranasally administered.
